# EUROPEAN PATENT APPLICATION

(11) **EP 4 692 110 A1**
(43) Date of publication of application: **11.02.2026**
(21) Application number: 24781122.7
(22) Date of filing: 21.03.2024
(51) Int. Cl.: C07K 14/00, A61P 3/04, A61K 8/64, A23L 33/18, A61K 38/00, A61P 3/00

(54) **PEPTIDE HAVING LIPOLYTIC EFFECT, AND USE THEREOF**

(30) Priority: 27.03.2023 KR 20230040066; 19.10.2023 KR 20230140081
(71) Applicant: Ninebiopharm Co., Ltd., Suwon-si Gyeonggi-do 16229 (KR)
(72) Inventor: RHIM, Jiheon, Suwon-si Gyeonggi-do 16229 (KR); KANG, Hae Yeong, Suwon-si Gyeonggi-do 16229 (KR); LEE, Rira, Suwon-si Gyeonggi-do 16229 (KR); KIM, Jae-Hwan, Suwon-si Gyeonggi-do 16229 (KR)
(74) Representative: Cabinet Becker et Associés
(86) International application number: PCT/KR2024/003571
(87) International publication number: WO 2024/205131

(57) **Abstract**

The present disclosure relates to a peptide having a lipolytic effect, and more particularly, to a composition for preventing, ameliorating, or treating obesity, the composition including the peptide. The peptide having a lipolytic effect according to the present disclosure is highly effective in terms of reducing intracellular lipid droplets, reducing cellulite, ameliorating skin roughness, and decreasing the length of the dermal and subcutaneous fat layer boundary, and thus can be widely used in the fields of preventing, ameliorating, or treating obesity.

## Description

### [Technical Field]

The present disclosure relates to a peptide having a lipolytic effect, and more particularly, to a composition for preventing, ameliorating, or treating obesity including the peptide.

### [Background Art]

Recently, in Korea, due to economic growth and westernization of diet, the intake of fat obtained from food has increased, and metabolic diseases such as obesity, diabetes, hyperlipidemia, hypertension, arteriosclerosis, and fatty liver are on the rise due to lack of exercise, and the like. In addition, the obesity not only harms the aesthetic appearance of young people who prefer a slim body, but also causes various diseases as obesity persists.

Current therapeutic agents for treating obesity may be broadly divided into drugs that affect appetite by acting on the central nervous system and drugs that act on the gastrointestinal tract to inhibit absorption. As the drugs that act on the central nervous system, drugs such as fenfluramine and dexfenfluramine that inhibit the serotonin (5-HT) nervous system, drugs such as ephedrine and caffeine through the noradrenergic nervous system, and recently, drugs such as sibutramine that act simultaneously on the serotonin and noradrenergic nervous systems to inhibit obesity, have been marketed according to each mechanism. In addition, orlistat and the like, which are approved as an obesity therapeutic agent that acts on the gastric/intestinal tract to inhibit the obesity and inhibits intestinal lipase to reduce fat absorption, have been used as a representative drug. However, among drugs that have been previously used, drugs such as fenfluramine have recently been banned due to side effects such as primary pulmonary hypertension and heart valve lesions, and other drugs also have problems such as decreased blood pressure and lactic acidosis, which cannot be used in patients with heart failure or kidney disease.

### [Disclosure]

### [Technical Problem]

Accordingly, the present inventors developed Regentide-LLP053 while searching for a novel substance for treating obesity, and confirmed a lipolytic effect thereof, and then completed the present disclosure.

Therefore, an object of the present disclosure is to provide a peptide represented by an amino acid sequence represented by SEQ ID NO: 1.

Another object of the present disclosure is to provide a composition for preventing, ameliorating or treating obesity, including a peptide represented by an amino acid sequence represented by SEQ ID NO: 1.

Yet another object of the present disclosure is to provide a slimming composition including a peptide represented by an amino acid sequence represented by SEQ ID NO: 1.

Still another object of the present disclosure is to provide a composition for reducing or ameliorating cellulite, including a peptide represented by an amino acid sequence represented by SEQ ID NO: 1.

Still another object of the present disclosure is to provide a method for preventing or treating obesity, including administering a peptide represented by an amino acid sequence represented by SEQ ID NO: 1 to a subject in need thereof.

Still another object of the present disclosure is to provide a slimming method including administering a peptide represented by an amino acid sequence represented by SEQ ID NO: 1 to a subject in need thereof.

Still another object of the present disclosure is to provide a method for reducing or ameliorating cellulite including administering a peptide represented by an amino acid sequence represented by SEQ ID NO: 1 to a subject in need thereof.

### [Technical Solution]

To achieve the object, an aspect of the present disclosure provides a peptide represented by an amino acid sequence represented by SEQ ID NO: 1.

Another aspect of the present disclosure provides a cosmetic composition for preventing or ameliorating obesity, including a peptide represented by an amino acid sequence represented by SEQ ID NO: 1.

Yet another aspect of the present disclosure provides a pharmaceutical composition for preventing or treating obesity, including a peptide represented by an amino acid sequence represented by SEQ ID NO: 1.

Still another aspect of the present disclosure provides a food composition for preventing or ameliorating obesity, including a peptide represented by an amino acid sequence represented by SEQ ID NO: 1.

Still another aspect of the present disclosure provides a cosmetic composition for slimming, including a peptide represented by an amino acid sequence represented by SEQ ID NO: 1.

Still another aspect of the present disclosure provides a food composition for slimming, including a peptide represented by an amino acid sequence represented by SEQ ID NO: 1.

Still another aspect of the present disclosure provides a cosmetic composition for reducing or ameliorating cellulite, including a peptide represented by an amino acid sequence represented by SEQ ID NO: 1.

Still another aspect of the present disclosure provides a food composition for reducing or ameliorating cellulite, including a peptide represented by an amino acid sequence represented by SEQ ID NO: 1.

Still another aspect of the present invention provides a method for preventing or treating obesity, including administering a peptide represented by an amino acid sequence represented by SEQ ID NO: 1 to a subject in need thereof.

Still another aspect of the present invention provides a slimming method, including administering a peptide represented by an amino acid sequence represented by SEQ ID NO: 1 to a subject in need thereof.

Still another aspect of the present invention provides a method for reducing or ameliorating cellulite, including administering a peptide represented by an amino acid sequence represented by SEQ ID NO: 1 to a subject in need thereof.

### [Advantageous Effects]

According to the present disclosure, the peptide having the lipolytic effect is excellent in reducing intracellular lipid droplets, reducing cellulite, ameliorating skin roughness, and decreasing the length of the dermal and subcutaneous fat layer boundary, and thus can be widely used in the fields of preventing, ameliorating, or treating obesity.

### [Description of Drawings]

FIG. 1 is a diagram showing results of evaluating the cytotoxicity of Regentide-LLP053 according to the present disclosure.
FIG. 2A is a diagram showing results of observing intracellular lipid droplets by administration of Regentide-LLP053 of the present disclosure using a confocal microscope.
FIG. 2B is a diagram showing results of analyzing the number and size of intracellular lipid droplets by administration of Regentide-LLP053 of the present disclosure.
FIG. 2C is a diagram showing results of analyzing the size of intracellular lipid droplets by administration of Regentide-LLP053 of the present disclosure, based on a control group.
FIG. 3A is a diagram showing results of observing intracellular lipid droplets according to a concentration of Regentide-LLP053 of the present disclosure using a confocal microscope.
FIG. 3B is a diagram showing results of analyzing the number and size of intracellular lipid droplets according to a concentration of Regentide-LLP053 of the present disclosure.
FIG. 3C is a diagram showing results of analyzing the size of intracellular lipid droplets according to a concentration of Regentide-LLP053 of the present disclosure, based on a control group.
FIG. 4 is a diagram showing results of evaluating the skin permeability of Regentide-LLP053 according to the present disclosure.
FIG. 5 is a diagram showing results of evaluating a lifting improvement effect (volume) of Regentide-LLP053 according to the present disclosure on the double chin area.
FIG. 6 is a diagram showing results of evaluating a lifting improvement effect (elevation volume) of Regentide-LLP053 according to the present disclosure on the double chin area.
FIG. 7 is a diagram showing results of evaluating a cellulite reduction effect of Regentide-LLP053 according to the present disclosure.
FIG. 8 is a diagram showing results of evaluating a skin roughness reduction effect of Regentide-LLP053 according to the present disclosure.
FIG. 9 is a diagram showing results of evaluating the length of the dermal and subcutaneous fat layer boundary of Regentide-LLP053 according to the present disclosure.

### [Best Mode]

Hereinafter, the present disclosure will be described in detail.

According to an aspect of the present disclosure, the present disclosure provides a peptide represented by an amino acid sequence represented by SEQ ID NO: 1.

In the present disclosure, the peptide refers to a linear molecule formed by binding amino acid residues to each other by peptide bonds. The peptide may be prepared according to a chemical synthesis method known in the art, preferably a solid-phase synthesis technique, but is not limited thereto.

In a specific embodiment of the present disclosure, the peptide represented by the amino acid sequence represented by SEQ ID NO: 1 preferably has an amine group (-NH₂) conjugated to a C-terminus, and the peptide conjugated with the amine group was referred to as 'Regentide-LLP053'. The Regentide-LLP053 may be represented by an amino acid represented by SEQ ID NO: 2. The Regentide-LLP053 has an amine group conjugated to the C-terminus of the peptide represented by the amino acid sequence represented by SEQ ID NO: 1, which may significantly improve stability within the tissue.

In a specific embodiment of the present disclosure, the peptide may have at least one activity selected from the group consisting of reducing intracellular lipid droplets, reducing cellulite, ameliorating skin roughness, and decreasing the length of the dermal and subcutaneous fat layer boundary.

The scope of the present disclosure includes functional equivalents of the peptide represented by the amino acid sequence represented by SEQ ID NO: 1.

In the present disclosure, the functional equivalents refer to peptides which have sequence homology (that is, identity) of at least 80% or more, preferably 90%, more preferably 95% or more with the amino acid sequence represented by SEQ ID NO: 1, for example, sequence homology of 80%, 81%, 82%, 83%, 84%, 85%, 86%, 87%, 88%, 89%, 90%, 91%, 92%, 93%, 94%, 95%, 96%, 97%, 98%, 99%, and 100%, as a result of addition, substitution or deletion of amino acids, and exhibit substantially homogeneous physiological activity as the peptide represented by the amino acid sequence represented by SEQ ID NO: 1. In addition, the peptide represented by the amino acid sequence represented by SEQ ID NO: 1 of the present disclosure includes not only proteins having its native amino acid sequence but also amino acid sequence variants thereof within the scope of the present disclosure. The variant of the peptide represented by the amino acid sequence represented by SEQ ID NO: 1 means a peptide having a sequence that is different from a natural amino acid sequence of the peptide by deletion, insertion, non-conservative or conservative substitution of one or more amino acid residues, or combinations thereof. Amino acid exchange in proteins and peptides without changing the activity of the molecule as a whole is known in the art. The peptide represented by the amino acid sequence represented by SEQ ID NO: 1 or variants thereof may be extracted naturally, synthesized, or prepared by a genetic recombination method based on a DNA sequence.

In addition, the sequence homology may be determined by a general standard method used to compare similar portions of amino acid sequences constituting the peptides. A computer program such as BLAST or FASTA aligns amino acids constituting two or more proteins so as to be optimally matched with each other (according to a full-length sequence of one or two sequences, or a predicted portion of one or two sequences). The program provides a default opening penalty and a default gap penalty and provides a scoring matrix such as PAM250 (standard scoring matrix) which may be used in association with the computer program. For example, the sequence homology represented by a percentage may be calculated as follows. The total number of identical matches is multiplied by 100 and then divided into a sum of the length of a longer sequence in a corresponding matched span and the number of gaps introduced into the longer sequence to align the two sequences.

In the present disclosure, the substantially homogeneous physiological activity means obesity treatment activity, and more specifically, at least one activity selected from the group consisting of reducing intracellular lipid droplets, reducing cellulite, ameliorating skin roughness, and decreasing the length of the dermal and subcutaneous fat layer boundary.

In addition, the range of the functional equivalents includes derivatives in which some chemical structures of constituting amino acids are modified while maintaining a basic backbone of the peptide represented by the amino acid sequence represented by SEQ ID NO: 1 and the obesity treatment activity. For example, the range of the functional equivalents includes structural modifications for changing the stability, storage, volatility, solubility or the like of the protein.

According to another aspect of the present disclosure, the present disclosure provides a composition for preventing, ameliorating or treating obesity, including a peptide represented by the amino acid sequence represented by SEQ ID NO: 1. The composition may be a cosmetic composition for preventing or ameliorating obesity; a pharmaceutical composition for preventing or treating obesity; or a food composition for preventing or ameliorating obesity.

In a specific embodiment of the present disclosure, the concentration of the peptide is preferably 0.1 to 1000 µM, more preferably 1 to 100 µM, and most preferably 10 to 20 µM.

When the composition according to the present disclosure is the cosmetic composition, the cosmetic composition of the present disclosure may further include conventional adjuvants and carriers, such as antioxidants, stabilizers, solubilizers, vitamins, pigments, fragrances, etc., which are conventionally used in cosmetic compositions, in addition to the active ingredients. For example, the cosmetic composition may further include auxiliary ingredients, such as glycerin, butylene glycol, polyoxyethylene hydrogenated castor oil, tocopheryl acetate, citric acid, panthenol, squalane, sodium citrate, and allantoin.

Since the cosmetic composition of the present disclosure is basically applied to the skin, the cosmetic composition may be prepared in any formulation that is commonly prepared by referring to cosmetic compositions in the art. For example, the cosmetic composition of the present disclosure may be formulated into solutions, suspensions, emulsions, pastes, gels, creams, lotions, powders, soap, surfactant-containing cleansing, oils, powder foundations, emulsion foundations, wax foundations, sprays, and the like, but is not limited thereto. More specifically, the cosmetic composition may be prepared in the formulation of a toner, a nourishing toner, a nourishing cream, a massage cream, an essence, an eye cream, a cleansing cream, a cleansing foam, a cleansing water, a mask pack, a spray, or a powder.

When the formulation of the present disclosure is the paste, cream, or gel, the carrier ingredient may include animal oils, vegetable oils, wax, paraffin, starch, tragacanth, cellulose derivatives, polyethylene glycol, silicone, bentonite, silica, talc, zinc oxide or the like.

When the formulation of the present disclosure is the powder or spray, the carrier ingredient may include lactose, talc, silica, aluminum hydroxide, calcium silicate, polyamide powder, or the like, and particularly, in the case of the spray, the carrier ingredient may further include a propellant such as chlorofluorohydrocarbon, propane/butane or dimethyl ether.

When the formulation of the present disclosure is the solution or emulsion, the carrier ingredient may include a solvent, a solubilizing agent, an emulsifying agent, or the like, and specifically, the carrier ingredient may include water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, fatty acid ester of sorbitan, or the like.

When the formulation of the present disclosure is the suspension, the carrier ingredient may include liquid diluents such as water, ethanol, and propylene glycol; suspensions such as ethoxylated isostearyl alcohol, polyoxyethylene sorbitol ester and polyoxyethylene sorbitan ester; microcrystalline cellulose, aluminum metahydroxide, bentonite, agar, tragacanth, or the like.

When the formulation of the present disclosure is the surfactant-containing cleansing, the carrier ingredient may include aliphatic alcohol sulfate, aliphatic alcohol ether sulfate, sulfosuccinic acid monoester, isethionate, imidazolinium derivatives, methyltaurate, sarcosinate, fatty acid amide ether sulfate, alkylamido betaine, aliphatic alcohol, fatty acid glyceride, fatty acid diethanolamide, vegetable oil, lanolin derivatives, ethoxylated glycerol fatty acid ester, or the like.

When the composition of the present disclosure is the pharmaceutical composition, the composition may be formulated in various forms and used according to conventional methods. For example, the composition may be prepared in oral formulations, such as powders, granules, tablets, capsules, suspensions, emulsions, syrups, and the like, and formulated and used in the form of external preparations, suppositories, and sterile injection solutions. However, it is most preferable that the composition of the present disclosure may be provided in the form of skin external preparations. Specifically, the composition may be used in the form of liquids, ointments, creams, lotions, sprays, patches, gels, aerosols, or the like.

In addition, according to each formulation, the composition may further include pharmaceutically acceptable carriers, excipients and diluents. In addition, the composition may be formulated and used in the form of powders, granules, tablets, capsules, suspensions, emulsions, syrups, external preparations such as aerosols, and sterile injection solutions according to conventional methods, and preferably, may have formulations of creams, gels, patches, sprays, ointments, oral preparations, lotions, liniments, pastes, or cataplasmas. For example, in the case of skin external preparations used locally on the corresponding area, the skin external preparations may include conventional additives, such as preservatives, solvents that aid drug penetration, emollients in the case of ointments and creams, and may contain conventional carriers, such as ethanol or oleyl alcohol. Suitable formulations known in the art are preferably used with those disclosed in the literatures, but are not limited thereto.

The carriers, the excipients, and the diluents may include lactose, dextrose, sucrose, oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oils, etc. In the case of preparing or formulating the pharmaceutical composition, the formulations may be prepared by using diluents or excipients, such as a filler, an extender, a binder, a wetting agent, a disintegrating agent, and a surfactant, which are generally used. Solid formulations for oral administration include tablets, pills, powders, granules, capsules, and the like, and these solid formulations may be prepared by mixing at least one or more excipients, for example, starch, calcium carbonate, sucrose, lactose, gelatin, and the like. Further, lubricants such as magnesium stearate and talc may be used in addition to simple excipients. Liquid formulations for oral administration may correspond to a suspension, an oral liquid, an emulsion, a syrup, and the like, and may include various excipients, for example, a wetting agent, a sweetener, an aromatic agent, a preserving agent, and the like, in addition to water and liquid paraffin which are commonly used as simple diluents. Formulations for parenteral administration include sterilized aqueous solutions, non-aqueous solvents, suspensions, emulsions, lyophilized agents, suppositories, and the like. As the non-aqueous solution and the suspension, propylene glycol, polyethylene glycol, vegetable oil such as olive oil, injectable ester such as ethyl oleate, and the like may be used. As a base of the suppository, witepsol, macrogol, tween 61, cacao butter, laurinum, glycerogelatin, and the like may be used. The ingredients may be added independently or in combination to an active ingredient, that is, an Ambrosia trifida supercritical extract.

As used herein, the administering means providing the pharmaceutical composition of the present disclosure to a subject by any suitable method.

The pharmaceutical composition of the present disclosure may be administered in a therapeutically effective amount, which is an amount of the active ingredient or pharmaceutical composition that induces biological or medical responses in tissues, animal or human considered by researchers, veterinarians, physicians or other clinicians, i.e., an amount that induces amelioration of symptoms of diseases or disorders to be treated. It is apparent to those skilled in the art that the therapeutically effective dose and the frequency of administration for the pharmaceutical composition of the present disclosure will vary depending on a desired effect. Therefore, an optimal dose to be administered may be easily determined by those skilled in the art, and may be adjusted according to various factors including a type of disease, the severity of a disease, the contents of active ingredients and other ingredients contained in the composition, a type of formulation, age, body weight, general health condition, sex and diet of a patient, an administration time, a route of administration, a secretion rate of a composition, duration of treatment, and drugs to be used concurrently.

The pharmaceutical composition of the present disclosure may be administered in an amount of 1 to 10,000 mg/kg/day, preferably 1 to 200 mg/kg/day, and may also be administered once a day or divided into several doses.

The food composition according to the present disclosure may be used as health functional foods, food additives or dietary supplements.

When the peptide represented by the amino acid sequence represented by SEQ ID NO: 1 is used as a food additive, the peptide may be used appropriately according to a conventional method, such as adding the mixture as it is or mixing and using the mixture with other foods or food ingredients.

In addition, the mixing amount of the peptide represented by the amino acid sequence represented by SEQ ID NO: 1 may be appropriately changed depending on the purpose of use (prevention, health or therapeutic treatment), and is included in an amount of preferably 0.01 to 95 wt%, and more preferably 1 to 80 wt% with respect to the total weight of the food composition. When the content is less than 0.01 wt%, the effect may be minimal, and when the content exceeds 95 wt%, an effect increase rate compared to the amount used may be low, which may be uneconomical.

As a specific example, when preparing foods or beverages, the peptide represented by the amino acid sequence represented by SEQ ID NO: 1 of the present disclosure is added in an amount of 15 wt% or less, preferably 10 wt% or less, with respect to the raw material. However, in the case of long-term intake for the purpose of health and hygiene or for the purpose of health control, the peptide may be added in an amount below the above range, and since there are no safety issues, the active ingredient may also be used in an amount above the above range.

There is no particular limitation in the type of food, but examples of the food which may be added with the peptide represented by the amino acid sequence represented by SEQ ID NO: 1 of the present disclosure include meat, sausage, bread, chocolate, candy, snacks, confectionery, pizza, ramen, other noodles, gum, dairy products including ice cream, various soups, beverages, tea, drinks, alcoholic beverages, vitamin complexes, etc., and include all health foods in a general meaning.

When the food composition of the present disclosure is prepared as beverages, like general beverages, the food composition may include additional ingredients such as various flavoring agents or natural carbohydrates. The natural carbohydrates may be used with monosaccharides such as glucose and fructose; disaccharides such as maltose and sucrose; natural sweeteners such as dextrin and cyclodextrin; synthetic sweeteners such as saccharin and aspartame; and the like. The natural carbohydrates are included in an amount of 0.01 to 10 wt%, preferably 0.01 to 0.1 wt%, with respect to the total weight of the food composition of the present disclosure.

The food composition of the present disclosure may include various nutrients, vitamins, electrolytes, flavoring agents, coloring agents, pectic acid and salts thereof, alginic acid and salts thereof, organic acid, a protective colloidal thickener, a pH adjusting agent, a stabilizer, a preservative, glycerin, alcohol, a carbonic acid agent used in carbonated beverages, etc., and may include fruit pulp for the preparation of natural fruit juices, fruit juice drinks, and vegetable drinks, but is not limited thereto. These ingredients may be used independently or in combination. The ratio of the additives is not particularly limited, but is preferably included in the range of 0.01 to 0.1 wt% with respect to the total weight of the food composition of the present disclosure.

In the case of long-term intake for the purpose of health and hygiene or health control, the food composition of the present disclosure may be taken for a long period of time because there is no problem in terms of safety.

According to another aspect of the present disclosure, the present disclosure provides a slimming composition including a peptide represented by an amino acid sequence represented by SEQ ID NO: 1. The slimming composition may be a cosmetic composition or a food composition.

According to yet another aspect of the present disclosure, the present disclosure provides a composition for reducing or ameliorating cellulite including a peptide represented by an amino acid sequence represented by SEQ ID NO: 1. The composition for reducing or ameliorating cellulite may be a cosmetic composition or a food composition.

In a specific embodiment of the present disclosure, the slimming is preferably at least one selected from the group consisting of reducing intracellular lipid droplets, reducing cellulite, ameliorating skin roughness, and decreasing the length of the dermal and subcutaneous fat layer boundary.

As used herein, the term 'slimming' refers to a phenomenon of weight loss or cellulite reduction.

In the present disclosure, the cellulite is also called edemato-fibrosclerotic panniculitis (EFSP) in medical terms, and causes congestion and edema due to microvascular-lymphatic circulation failure in a local subcutaneous area, and as fibrosis progresses, the fibrous septa are thickened and elasticity is lost, and while pulling the skin, the skin becomes bumpy. The cellulite occurs more frequently in areas where local fat is excessively accumulated, but is not necessarily accompanied by overweight or obesity.

That is, the slimming; and the reduction or amelioration of cellulite are not necessarily associated with obesity and have separate meanings in themselves.

According to yet another aspect of the present disclosure, the present disclosure provides a method for preventing or treating obesity, including administering a peptide represented by an amino acid sequence represented by SEQ ID NO: 1 to a subject in need thereof.

In a specific embodiment of the present disclosure, the subject may be a subject expected to develop obesity; a subject suffering from obesity; or a subject completely cured of obesity, but is not limited thereto.

According to still another aspect of the present disclosure, the present disclosure provides a method for preventing or treating obesity, including administering a peptide represented by an amino acid sequence represented by SEQ ID NO: 1 to a subject in need thereof.

In a specific embodiment of the present disclosure, the subject may be a subject expected to develop obesity; a subject suffering from obesity; or a subject completely cured of obesity, but is not limited thereto.

According to still another aspect of the present disclosure, the present disclosure provides a slimming method, including administering a peptide represented by an amino acid sequence represented by SEQ ID NO: 1 to a subject in need thereof. Further, the present disclosure provides a method for reducing or ameliorating cellulite, including administering a peptide represented by an amino acid sequence represented by SEQ ID NO: 1 to a subject in need thereof.

In a specific embodiment of the present disclosure, the subject may be a subject in need of slimming or cellulite reduction. More particularly, the subject in need of slimming may be a subject in need of at least one selected from the group consisting of reducing intracellular lipid droplets, reducing cellulite, ameliorating skin roughness, and decreasing the length of the dermal and subcutaneous fat layer boundary.

The aforementioned slimming and reduction or amelioration of cellulite are not necessarily associated with obesity and have separate meanings in themselves.

Duplicated contents will be omitted in consideration of the complexity of the present specification, and terms not defined otherwise herein have the meanings commonly used in the art to which the present disclosure pertains.

### [Modes]

Hereinafter, the present disclosure will be described in more detail through Examples. These Examples are just illustrative of the present disclosure, and it will be apparent to those skilled in the art that the scope of the present disclosure is not to be construed as being limited by these Examples.

### Example 1. Peptide synthesis

A peptide with a lipolytic effect was synthesized. The synthesized peptide was represented by an amino acid sequence represented by SEQ ID NO: 1, and an amine group (-NH₂) was conjugated to a C-terminus of each peptide for stability in tissues. The peptide with an amine group conjugated to the C-terminus was represented by SEQ ID NO: 2, and the peptide with the amine group conjugated to the C-terminus was referred to as 'Regentide-LLP053', respectively.

**[Table 1]**

| Name | Amino acid sequence |
|---|---|
| Regentide-LLP053 (SEQ ID NO: 1) | SPKMVQGSGCFGRKMDRISSSSGLGC |
| Regentide-LLP053 (SEQ ID NO: 2) | SPKMVQGSGCFGRKMDRISSSSGLGC-NH₂ |

### Example 2. Evaluation of cytotoxicity of Regentide-LLP053

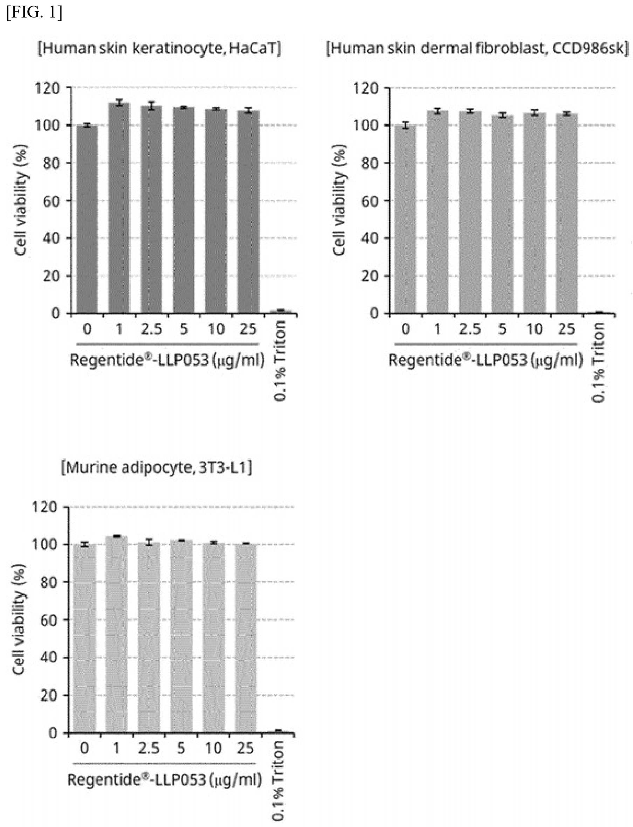

To confirm cytotoxicity of Regentide-LLP053, the cytotoxicity was measured in human epidermal keratinocytes HaCaT, human fibroblasts CCD-986Sk, and mouse preadipocytes 3T3-L1. All three cells were dispensed in a 96-well plate at a density of 3 × 10³ cells/well and incubated in a medium containing 10% fetal bovine serum (FBS) for 24 hours under cell culture conditions (37°C, 5% CO₂). After incubation, the cells were treated with 0, 1, 2.5, 5, 10, and 25 µg/ml of Regentaid-LLP053 in a FBS-free medium and incubated for 48 hours. In addition, a positive control group was treated with 0.1% Triton-X and incubated for 48 hours. Thereafter, 10 µl of a CCK8 reaction solution (Dojindo Molecular Technologies, Japan) was added per well and reacted for 4 hours, and then the absorbance was measured at 450 nm. A mean absorbance value for each sample group was calculated, and cell viability was evaluated by comparing the mean absorbance value with an absorbance value of cells which were not treated with Regentide-LLP053 as a control group. The results of evaluating the cell viability were shown in FIG. 1.

As shown in FIG. 1, it was confirmed that there was no cytotoxicity in the group treated with Regentide-LLP053 compared to the group treated with 0.1% Triton-X as the positive control group.

### Example 3. Lipolytic effect of Regentide-LLP053

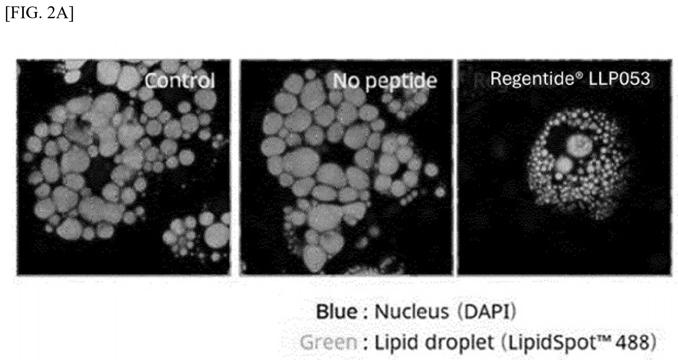

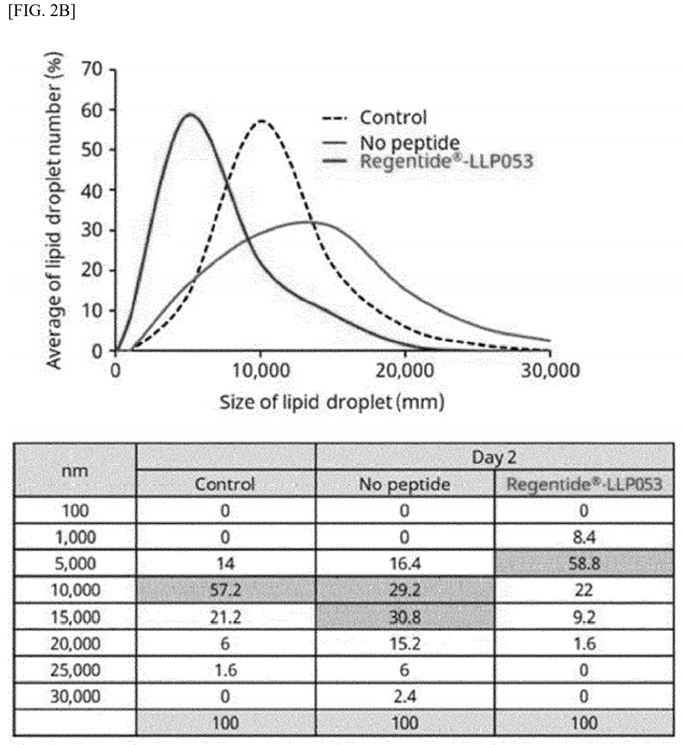

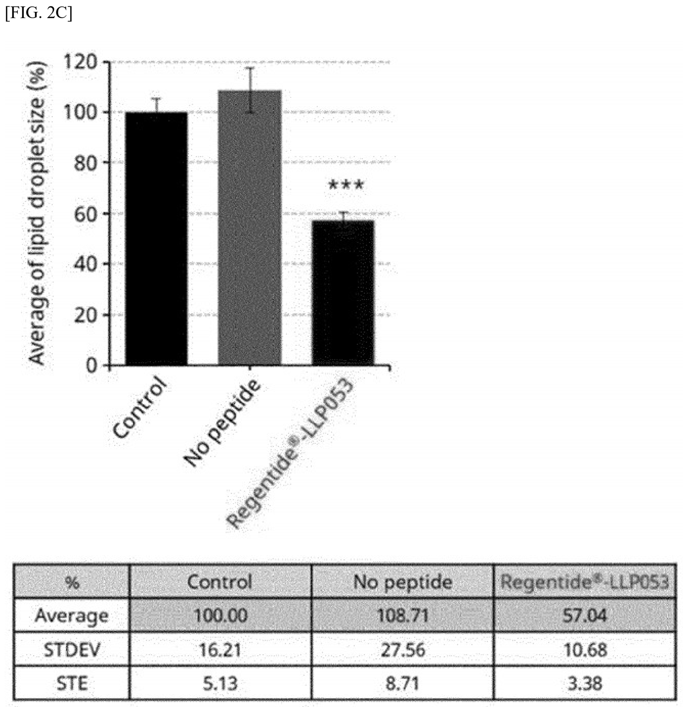

3T3-L1 cells (Korea Cell Line Bank, Korea) as preadipocytes were incubated under cell culture conditions (37°C, 5% CO₂) in a high-glucose DMEM (Sigma-Aldrich, MA, USA) medium supplemented with 10% FBS and a 1% (v/v) antibiotic/antimycotic solution. To differentiate the 3T3-L1 cells as preadipocytes into adipocytes, the cells were dispensed at 1 × 10⁵ cells/well in a 6-well plate, and then when the cell density was approximately 85 to 90%, adipose differentiation was induced using 10% FBS and a preadipose differentiation kit (3T3-L1 Differentiation Kit; Abcam, UK), and the culture medium was replaced every two days with DMEM F-12 Ham (MDI) containing 1.5 µg/mL of insulin. After about 10 days, it was confirmed that 90% or more of the cells were transformed into round-shaped mature adipocytes. After treating adipose differentiation-induced cells with Regentide-LLP053 at a concentration of 10 µM for 2 days, lipid droplets were identified using a fluorescent compound (LipidSpot^{™} 488 Lipid Droplet Stains; Biotium, USA) that selectively stained intracellular lipid droplets to confirm the lipolytic effect. To stain the lipid droplets with the fluorescent compound, the cells were rinsed with 1x phosphate-buffered saline (PBS) and immobilized with a fixative (4% paraformaldehyde solution; PFA, Pobio Korea, Korea) for 15 minutes at room temperature. The cells were washed three times with 1x PBS and then treated with the fluorescent compound and stained for 30 minutes at room temperature. After staining, the cells were washed once with 1x PBS and then encapsulated with a cell nuclei staining solution (Fluoroshield^{™} with DAPI; Sigma-Aldrich, USA). The lipid droplets in the stained cells were observed using a confocal laser scanning microscope (Carl Zeiss, Oberkochen, Germany). The lipolytic effect analysis was shown in a graph by measuring the number and size of intracellular lipid droplets using the Zen 3.3 blue edition (Carl Zeiss, Germany) program on images captured with a confocal fluorescence microscope. The sizes and number of lipid droplets were confirmed using the Zen 3.3 blue edition program. The results of observing the intracellular lipid droplets using a confocal fluorescence microscope were shown in FIG. 2A, and the results of analyzing the number and size of intracellular lipid droplets were shown in FIGS. 2B and 2C, respectively.

As shown in FIG. 2A, it was confirmed that the size and number of lipid droplets were decreased in the group treated with 10 µM of Regentaid-LLP053 two days after treated with Regentaid-LLP053 compared to the control group (Control) and an experimental group not treated with Regentaid-LLP053 (No peptide).

As shown in FIG. 2B, it was confirmed that the lipid droplets of the control group (Control) were contained 57.2% at the size of 10,000 nm, and the lipid droplets of the experimental group (No peptide) were contained 29.2% at the size of 10,000 nm, and 30.8% at the size of 15,000 nm. On the other hand, in the case of the group treated with Regentide-LLP053, the lipid droplets were contained 58.8% at the size of 5,000 nm, and thus it was confirmed that the size of lipid droplets was reduced by Regentide-LLP053.

As shown in FIG. 2C, it was confirmed that when the lipid droplet size of the control group (Control) was considered 100%, the lipid droplet size of the experimental group (No peptide) that was not treated with Regentaid-LLP053 after adipose differentiation was 108.71%, whereas the lipid droplet size of the group treated with Regentaid-LLP053 was decreased to 57.04%. As a result, the lipolytic effect of Regentide-LLP053 was confirmed.

### Example 4. Lipolytic effect of Regentide-LLP053 by concentration

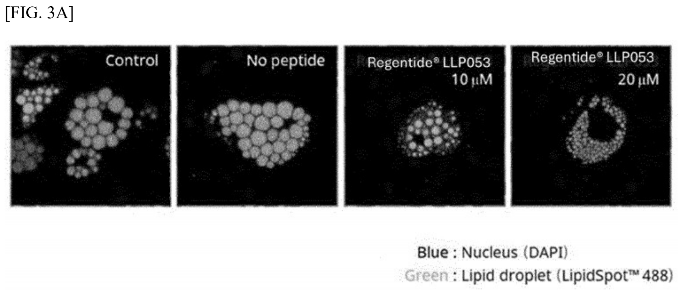

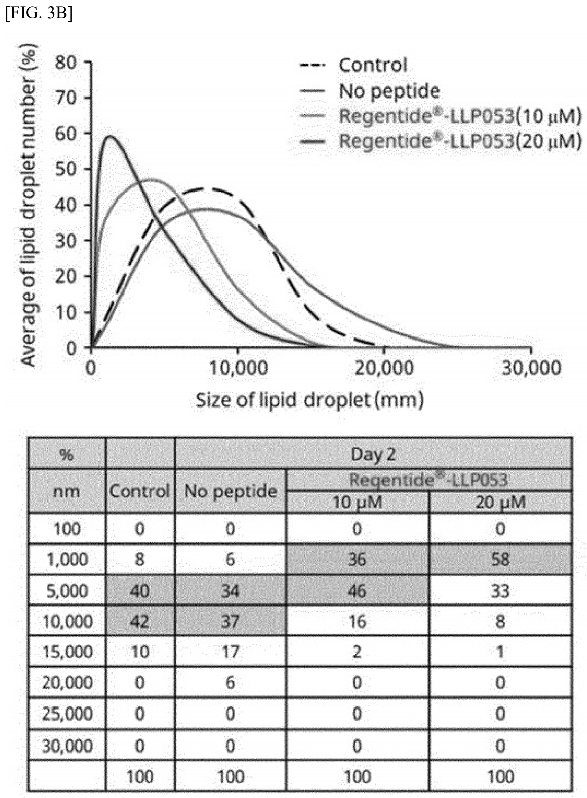

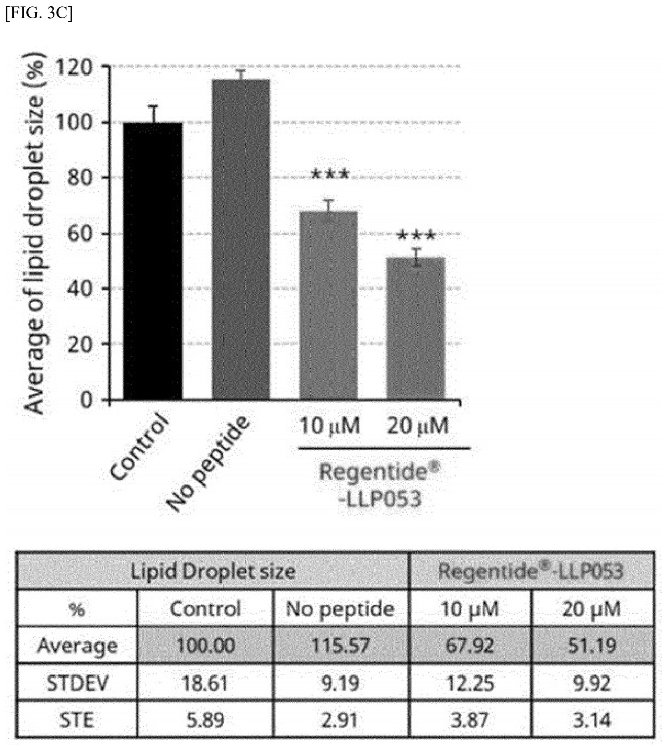

In the same manner as in Example 3, preadipocytes 3T3-L1 were differentiated into adipocytes, and then treated with Regentide-LLP053 by concentrations and incubated for 2 days. Thereafter, intracellular lipid droplets were observed using a confocal fluorescence microscope, and the size and number of lipid droplets were confirmed using the Zen 3.3 blue edition program. The results of observing the intracellular lipid droplets using the confocal fluorescence microscope were shown in FIG. 3A, and the results of analyzing the number and size of intracellular lipid droplets were shown in FIGS. 3B and 3C, respectively.

As shown in FIG. 3A, it was confirmed that the size and number of lipid droplets were decreased in the group treated with 10 µM and 20 µM of Regentaid-LLP053 two days after being treated with Regentaid-LLP053, compared to the control group (Control) and the experimental group not treated with Regentaid-LLP053 (No peptide).

As shown in FIG. 3B, it was confirmed that according to the size of lipid droplets, the number of lipid droplets was 40% at a size of 5,000 nm and 42% at a size of 10,000 nm in the control group (Control), and 34% at the size of 5,000 nm and 37% at the size of 10,000 nm in the experimental group (No peptide). On the other hand, it was confirmed that according to the concentration of Regentide-LLP053, the size of lipid droplets was further reduced to 36% at the size of 1,000 nm and 46% at the size of 5,000 nm in the group treated with 10 µM of Regentaid-LLP053, and 58% at the size of 1,000 nm in the group treated with 20 µM of Regentaid-LLP053.

As shown in FIG. 3C, it was confirmed that when the lipid droplet size of the control group (Control) was 100%, the experimental group (No peptide) that was not treated with Regentide-LLP053 after adipose differentiation was 115.57%. On the other hand, it was confirmed that according to a concentration, the size of lipid droplets was decreased to 67.92% in the group treated with 10 µM of Regentide-LLP053 and 51.19% in the group treated with 20 µM of Regentide-LLP053. As a result, it was confirmed that the lipolytic effect was increased as the concentration of Regentide-LLP053 was increased.

### Example 5. Confirmation of skin permeability of Regentide-LLP053

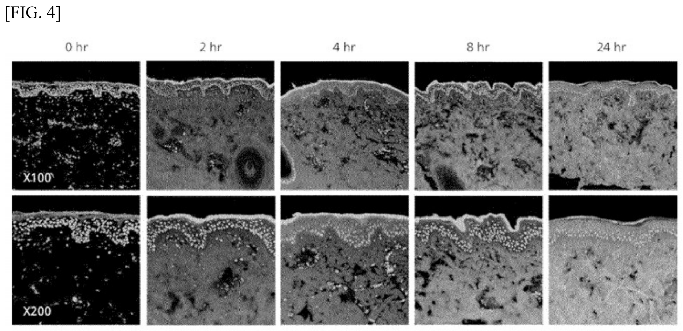

To determine the skin permeability of Regentide-LLP053, 30 µL of fluorescence (FITC)-labeled Regentide-LLP053 (1 mg/mL) was applied to the surface of research porcine skin (Micropig^{®} Franz cell membrane (FCM; APURES, Korea)) with a size of 1 cm × 1 cm × 500 µm and then treated for 0, 2, 4, 8, and 24 hours. After treatment, in order to prepare frozen sections, the samples were placed in a frozen tissue embedding agent (OCT Compound; FSC22 Frozen Section Compound, Leica Biosystems, USA), frozen in liquid nitrogen, and stored at - 80°C. The frozen sections were sectioned at 10 µm at -20°C using a cryostat microtome (Leica Biosystems, USA) as a sectioning device to prepare living tissue sections for frozen sections. The prepared frozen sections were treated with a cell nucleus staining solution (Fluoroshield^{™} with DAPI; Sigma-Aldrich, USA) and then the skin permeability was identified using a confocal fluorescence microscope (Confocal laser scanning microscope, Carl Zeiss, Oberkochen, Germany), and the results were shown in FIG. 4.

As shown in FIG. 4, it was confirmed that the skin permeability was shown from 2 hours after treatment with fluorescence-labeled Regentaid-LLP053, and the skin permeability increased over time.

### Example 6. Measurement (volume) of lifting improvement effect on double chin area

In order to confirm a clinical effect of Regentide-LLP053 on lipolytic ability, a lifting improvement effect of a liquid formulation prescribed with a composition on the double chin area was requested and identified by an authorized certification agency. A composition for lipolysis and cellulite improvement containing Regentide-LLP053 of Table 1 above was prepared as a liquid type product. The composition of the liquid type product was as shown in Table 2 below.

**[Table 2]**

| Raw material name (ICID Name) | Content (%) |
|---|---|
| Water | 97.5142 |
| 1,2-Hexanediol | 1.6000 |
| Sodium Chloride | 0.8856 |
| Regentide-LLP053(Sh-Oligopeptide-111 SP Amide) | 0.0002 |
| Total | 100 |

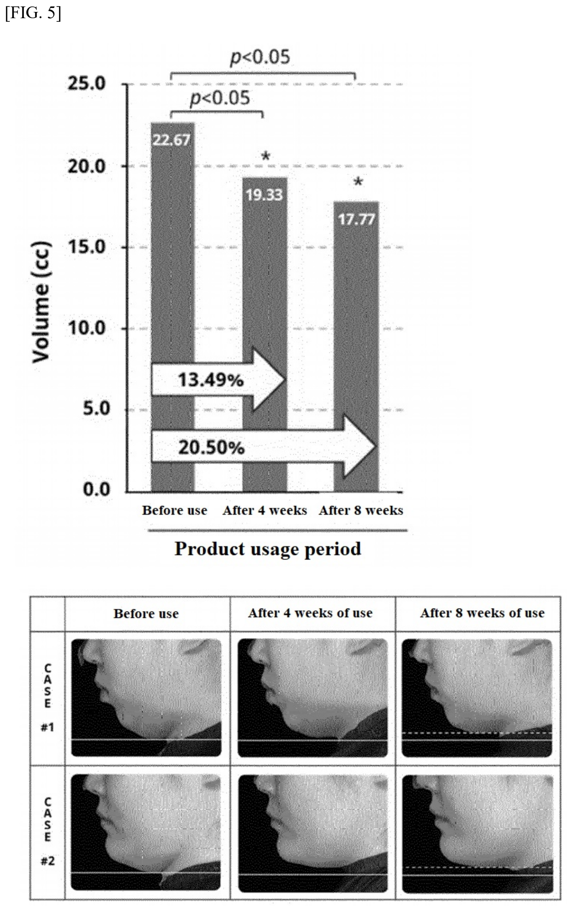

Test subjects who participated in the human application test were 21 healthy adults aged 40.62 ± 11.40 years. The test subjects applied an appropriate amount of a test product to the test area twice a day for 8 weeks, and gently massaged the skin as if pulled away from the muscles, and the lifting improvement effect on the double chin area was evaluated through device measurement. In order to set the same measurement conditions for the test subjects to evaluate the lifting improvement of the double chin area, the test area was kept clean and dry, and the test was conducted under constant temperature and humidity conditions (room temperature 20 to 24°C, humidity 40 to 60%) with no air movement and no direct sunlight. The test area was the facial area (double chin area), and the test area was captured using Vectra XT (Canfield Sci, USA) and then analyzed using Vectra Analysis Module (VAM; Canfield Sci, USA). The analysis variable was volume, and it was meant that as the measured value was decreased, the double chin lifting was improved. Statistical analysis was performed using SPSS statistics, and the test results were subjected to statistical analysis after performing a normality test. As a result of the analysis, when the significance probability was p < 0.05 at a 95% confidence interval, it was determined that there was an improvement effect due to the use of the test product. The results of confirming the lifting improvement effect on the double chin area were shown in Table 3 and FIG. 5.

**[Table 3]**

| (Unit: cc) | | | | |
|---|---|---|---|---|
| Timing | Analysis value (average ± standard deviation) | Improvement rate (%) | p-value | |
| Before product use (0 week) | 22.672 ± 7.243 | - | 0.000¹⁾ | - |
| After 4 weeks of product use | 19.327 ± 5.584 | 13.49 ± 9.30 | | 0.000²⁾ |
| After 8 weeks of product use | 17.767 ± 5.358 | 20.50 ± 10.23 | | 0.000³⁾ |
| 1) by Friedman's test (p < 0.05). | | | | |
| 2) by Wilcoxon signed rank test (p < 0.05). Before product use (0 week) vs After 4 weeks of product use | | | | |
| 3) by Wilcoxon signed rank test (p < 0.05). Before product use (0 week) vs After 8 weeks of product use | | | | |

As shown in Table 3 and FIG. 5, no adverse skin reaction due to the test product was observed during the evaluation period for the lifting improvement effect on the double chin area. As a result of analyzing the lifting effect on the double chin area by volume, the volume was statistically significantly reduced to 22.672 cc before product use (0 week), 19.327 cc after 4 weeks of product use, and 17.767 cc after 8 weeks of product use (p < 0.05). In addition, compared to before product use (0 week), the improvement rate was 13.49% after 4 weeks of product use and 20.50% after 8 weeks of product use.

### Example 7. Measurement (elevation volume) of lifting improvement effect on double chin area

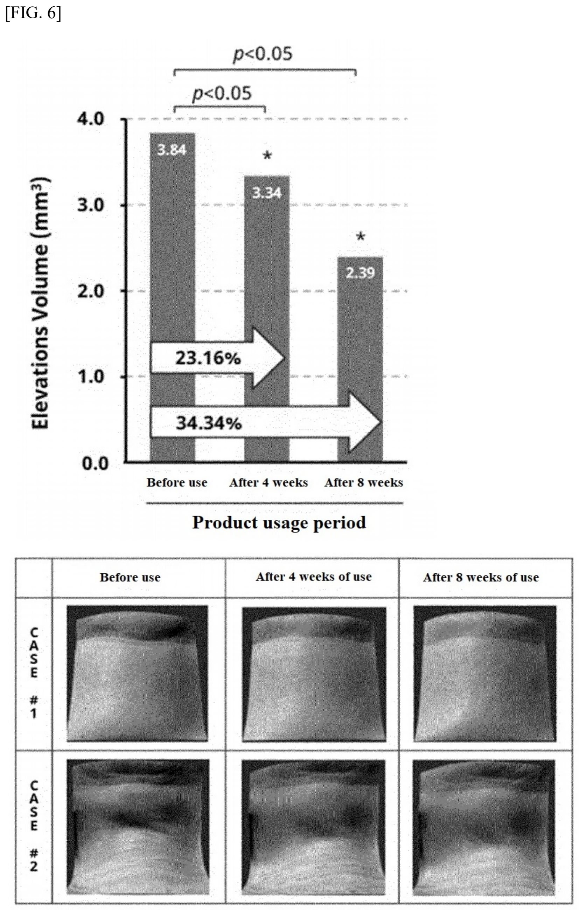

The lifting improvement effect (volume) on the double chin area was measured using the same test subjects and under the same measurement conditions as in Example 6. The test area was the facial area (double chin area), and captured and analyzed using Antera 3D (Miravex, Dublin, Ireland). The Antera 3D imaged the skin surface and applied the instrumental measurements using an analysis program provided by the instrument (Antera 3D software, Miravex, Dublin, Ireland). The captured image was used to measure the double chin volume using the Volumes mode of the analysis program, and the measurement variable was an elevation volume. It was meant that as the measurement value was decreased, the double chin lifting was improved. The results of analyzing the lifting effect on the double chin area were shown in Table 4 and FIG. 6.

**[Table 4]**

| (Unit: mm²) | | | | |
|---|---|---|---|---|
| Timing | Measurement value (average ± standard deviation) | Improvement rate (%) | p-value | |
| Before product use (0 week) | 3.8396 ± 4.9562 | - | 0.000¹⁾ | - |
| After 4 weeks of product use | 3.3357 ± 4.8907 | 23.16 ± 18.19 | | 0.001²⁾ |
| After 8 weeks of product use | 2.3936 ± 2.6470 | 34.34 ± 17.41 | | 0.000³⁾ |
| 1) by Friedman's test (p < 0.05). | | | | |
| 2) by Wilcoxon signed rank test (p < 0.05). Before product use (0 week) vs After 4 weeks of product use | | | | |
| 3) by Wilcoxon signed rank test (p < 0.05). Before product use (0 week) vs After 8 weeks of product use | | | | |

As shown in Table 4 and FIG. 6, the measurement value was decreased statistically significantly to 3.8396 mm² before product use (week 0), 3.3357 mm² after 4 weeks of product use, and 2.3936 mm² after 8 weeks of product use (p < 0.05). In addition, compared to before product use (0 week), the improvement rate was 23.16% after 4 weeks of product use and 34.34% after 8 weeks of product use.

### Example 8. Measurement of cellulite reduction effect

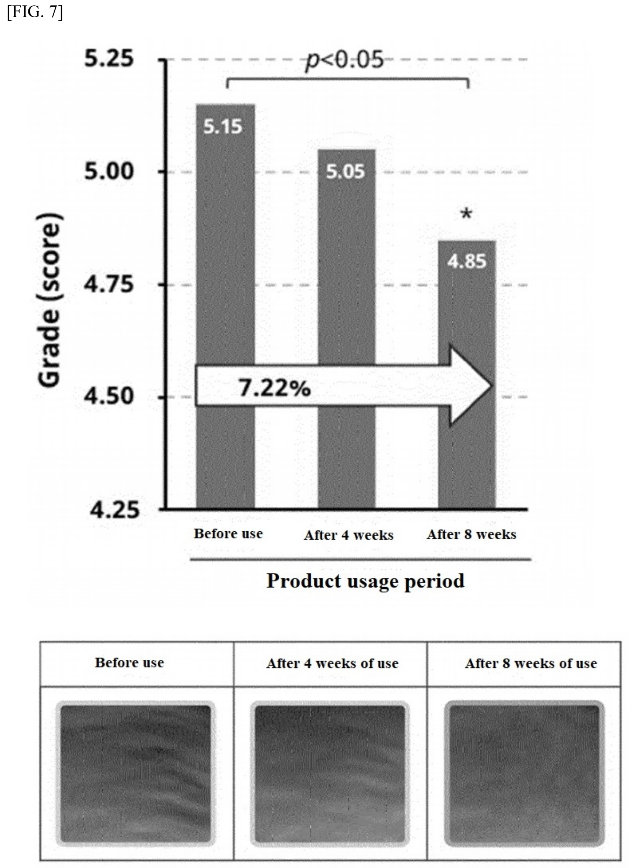

In order to confirm a clinical effect of Regentide-LLP053 on cellulite reduction efficacy, a cellulite reduction effect of a liquid formulation prescribed with a composition on the thigh area was requested and identified by an authorized certification agency. A composition for lipolysis and cellulite improvement containing Regentide-LLP053 of Table 1 above was prepared as a liquid type product (Table 2). Test subjects who participated in the human application test were 20 healthy adult women aged 36.00 ± 9.41 years, and persons with a body mass index (BMI) of 18 or higher and a cellulite assessment grade of 1 or higher were selected as the test subjects. The test subjects applied an appropriate amount of the test product to the test area once a day for 8 weeks, gently grasped the skin between the thumb and index fingers, and then massaged the skin as if being gently pulled from the muscles. The cellulite reduction effect was evaluated by visual evaluation, skin roughness evaluation, and measurement of the length of the dermis and subcutaneous fat layer boundary. In order to set the same measurement conditions for the test subjects, the test area was kept clean and dry, and the test was conducted under constant temperature and humidity conditions (room temperature 20 to 24°C, humidity 40 to 60%) with no air movement and no direct sunlight. The test area was the thigh area, and the test area was captured under the same conditions using a digital camera (DSLR EOS750D, CANON, USA) for visual evaluation, and after capturing the cellulite area, two evaluators evaluated the test area as 1 to 9 grades in a double-blind and randomized manner. Statistical analysis was performed using SPSS statistics, and the test results were subjected to statistical analysis after performing a normality test. As a result of the analysis, when the significance probability was p < 0.05 at a 95% confidence interval, it was determined that there was an improvement effect due to the use of the test product. The results of the visual evaluation of the cellulite reduction effect were shown in Table 5 and FIG. 7.

**[Table 5]**

| (Unit: score) | | | | |
|---|---|---|---|---|
| Timing | Measurement value (average ± standard deviation) | Improvement rate (%) | p-value | |
| Before product use (0 week) | 5.15 ± 1.53 | - | 0.030¹⁾ | - |
| After 4 weeks of product use | 5.05 ± 1.67 | 2.75 ± 10.91 | | 0.317²⁾ |
| After 8 weeks of product use | 4.85 ± 1.69 | 7.22 ± 13.82 | | 0.034³⁾ |
| 1) by Friedman's test (p < 0.05). | | | | |
| 2) by Wilcoxon signed rank test (p < 0.05). Before product use (0 week) vs After 4 weeks of product use | | | | |
| 3) by Wilcoxon signed rank test (p < 0.05). Before product use (0 week) vs After 8 weeks of product use | | | | |

As shown in Table 5 and FIG. 7, no adverse skin reaction due to the test product was observed during the evaluation period. As a result of visual evaluation, the grade was 5.15 before product use (0 week), 5.05 after 4 weeks of product use, and 4.85 after 8 weeks of product use, showing a statistically significant decrease after 8 weeks of product use (p < 0.05). In addition, compared to before product use (0 week), the improvement rate was 7.22% after 8 weeks of product use.

### Example 9. Evaluation of skin roughness

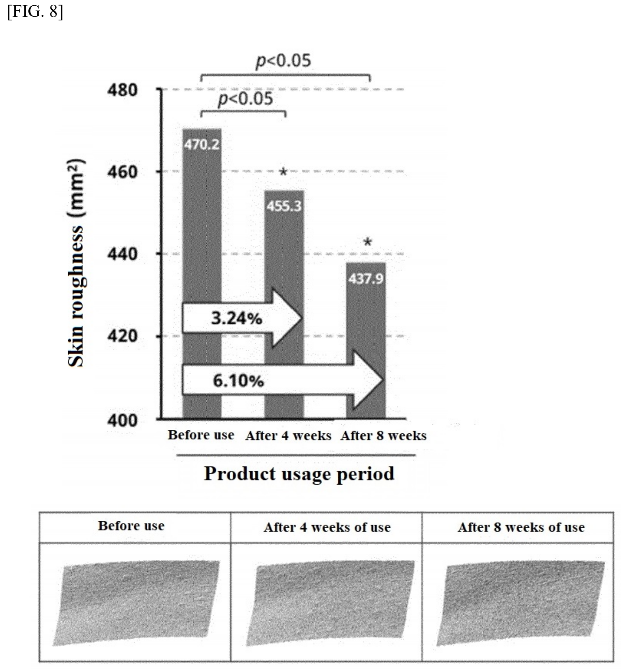

Skin roughness was evaluated under the same test subjects and the same measurement conditions as in Example 8. The test area was the thigh area, and the same area was captured using a 3D imaging device, PRIMOS CR (Canfield, USA), and then roughness parameters were analyzed using the provided software program. The analysis variable was the volume of skin roughness, and it was meant that as the analysis value was decreased, cellulite was decreased. The results of evaluating the skin roughness were shown in FIG. 8 and Table 6.

**[Table 6]**

| (Unit: mm³) | | | | |
|---|---|---|---|---|
| Timing | Measurement value (average ± standard deviation) | Improvement rate (%) | p-value | |
| Before product use (0 week) | 470.239 ± 90.198 | - | 0.001¹⁾ | - |
| After 4 weeks of product use | 455.252 ± 89.303 | 3.24 ± 3.09 | | 0.000²⁾ |
| After 8 weeks of product use | 437.880 ± 70.626 | 6.10 ± 6.78 | | 0.001³⁾ |
| 1) by Friedman's test (p < 0.05). | | | | |
| 2) by Wilcoxon signed rank test (p < 0.05). Before product use (0 week) vs After 4 weeks of product use | | | | |
| 3) by Wilcoxon signed rank test (p < 0.05). Before product use (0 week) vs After 8 weeks of product use | | | | |

As shown in FIG. 8 and Table 6, the volume was decreased statistically significantly to 470.239 mm³ before product use (week 0), 455.252 mm³ after 4 weeks of product use, and 437.880 mm³ after 8 weeks of product use (p < 0.05). In addition, compared to before product use (0 week), the improvement rate was 3.24% after 4 weeks of product use and 6.10% after 8 weeks of product use.

### Example 10. Evaluation of length of dermis and subcutaneous fat layer boundary

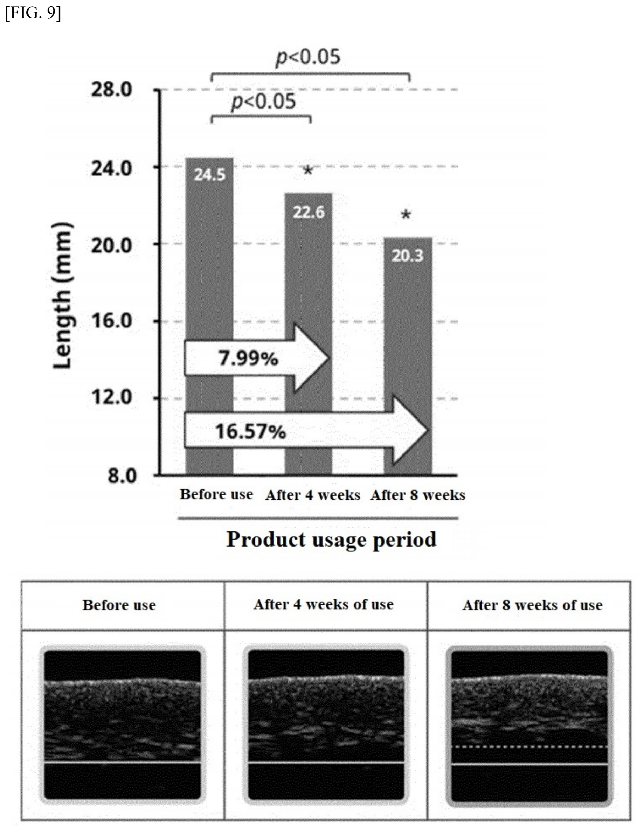

The length of the dermal and subcutaneous fat layer boundary was evaluated under the same test subjects and the same measurement conditions as in Example 8. The test area was the thigh area, and the length of the dermal and subcutaneous fat layer boundary of the test area was measured using a DUB Skinscanner (TPM, Germany). The analysis variable was Length, and it was meant that as the measurement value was decreased, cellulite was decreased. The results of evaluating the length of the dermal and subcutaneous fat layer boundary were shown in FIG. 9 and Table 7.

**[Table 7]**

| (Unit: mm) | | | |
|---|---|---|---|
| Timing | Measurement value (average ± standard deviation) | Improvement rate (%) | p-value |
| Before product use (0 week) | 24.499 ± 4.930 | - | - |
| After 4 weeks of product use | 22.621 ± 5.340 | 7.99 ± 8.69 | 0.001²⁾ |
| After 8 weeks of product use | 20.328 ± 4.841 | 16.57 ± 13.36 | 0.000³⁾ |
| 1) by Repeated Measures ANOVA, within-subject contrast test, @time (p < 0.05). Before product use (0 week) vs after 4 weeks of product use | | | |
| 2) by Repeated Measures ANOVA, within-subject contrast test, @time (p < 0.05). Before product use (0 week) vs after 8 weeks of product use | | | |

As shown in FIG. 9 and Table 7, as the measured result, the length of the dermal and subcutaneous fat layer boundary was statistically significantly decreased to 24.499 mm before product use (week 0), 22.621 mm after 4 weeks of product use, and 20.328 mm after 8 weeks of product use (p < 0.05). In addition, compared to before product use (0 week), the improvement rate was 7.99% after 4 weeks of product use and 16.57% after 8 weeks of product use.

As described above, specific parts of the present disclosure have been described in detail, and it will be apparent to those skilled in the art that these specific techniques are merely preferred embodiments, and the scope of the present disclosure is not limited thereto. Therefore, the substantial scope of the present disclosure will be defined by the appended claims and their equivalents.

## Claims

1. A peptide represented by an amino acid sequence represented by SEQ ID NO: 1.

2. The peptide of claim 1, wherein an amine group (-NH₂) is conjugated to a C-terminus.

3. The peptide of claim 1, wherein the peptide has at least one activity selected from the group consisting of reducing intracellular lipid droplets, reducing cellulite, ameliorating skin roughness, and decreasing a length of a dermal and subcutaneous fat layer boundary.

4. A cosmetic composition for preventing or ameliorating obesity, comprising a peptide represented by an amino acid sequence represented by SEQ ID NO: 1.

5. The cosmetic composition of claim 4, wherein the peptide has a concentration of 0.1 to 1000 µM.

6. A pharmaceutical composition for preventing or treating obesity, comprising a peptide represented by an amino acid sequence represented by SEQ ID NO: 1.

7. A food composition for preventing or ameliorating obesity, comprising a peptide represented by an amino acid sequence represented by SEQ ID NO: 1.

8. A cosmetic composition for slimming comprising a peptide represented by an amino acid sequence represented by SEQ ID NO: 1.

9. The cosmetic composition for slimming of claim 8, wherein the slimming is at least one selected from the group consisting of reducing intracellular lipid droplets, reducing cellulite, ameliorating skin roughness, and decreasing a length of a dermal and subcutaneous fat layer boundary.

10. A food composition for slimming comprising a peptide represented by an amino acid sequence represented by SEQ ID NO: 1.

11. A cosmetic composition for reducing or ameliorating cellulite comprising a peptide represented by an amino acid sequence represented by SEQ ID NO: 1.

12. A food composition for reducing or ameliorating cellulite comprising a peptide represented by an amino acid sequence represented by SEQ ID NO: 1.

13. A method for preventing or treating obesity, comprising administering a peptide represented by an amino acid sequence represented by SEQ ID NO: 1 to a subject in need thereof.
